# EUROPEAN PATENT APPLICATION

(11) **EP 4 786 572 A1**
(43) Date of publication of application: **05.08.2026**
(21) Application number: 25155289.9
(22) Date of filing: 31.01.2025
(51) Int. Cl.: C12M 1/26, B05B 17/06, B05B 17/00, C12M 1/34

(54) **MULTIFUNCTIONAL HYDRATION SYSTEM**

(71) Applicant: University of Galway, H91 TK33 Galway (IE)
(72) Inventor: O Brien, Timothy, Galway (IE); Jayasooriya, Dulan Hasantha, Galway (IE)
(74) Representative: Barker Brettell LLP

(57) **Abstract**

A device for generating liquid droplets comprising a body configured to hold a liquid, one or more piezoelectric actuators disposed within the body, and one or more piezoelectric sensors disposed on an underside of the body, wherein the piezoelectric sensors are configured to generate an electrical signal which is configured to activate the piezoelectric actuators. The one or more piezoelectric actuators are configured to oscillate at an ultrasonic frequency to atomise the liquid within the body to generate a plurality of droplets. A system for maintaining hydration of a biostructure, the system comprising the aforementioned device for generating liquid droplets, a hydration chamber, and a tubing configured to convey liquid droplets generated by the device into the hydration chamber. The hydration chamber comprising a chamber lid configured to fit over a printing plate, wherein the chamber lid comprises an inlet to receive the tubing.

## Description

The invention relates generally to a multipurpose system for generating liquid droplets and providing a controlled environment for three-dimensional bioprinting. More particularly, but not exclusively, the invention relates to a device for generating liquid droplets and a chamber for hydration of 3D-printed biomaterials using the generated liquid droplets.

### Background

3D bioprinting is a manufacturing process that uses 3D printing techniques to produce 3D structures composed of live cells and biocompatible "cell friendly" material known as "biomaterial". Most commonly, these two components are together generally referred to as "bioink". 3D bioprinting is used to produce live cell-harbouring 3D structures.

Unlike traditional 3D printing, which typically involves plastic or metal materials, 3D bioprinting uses biomaterial and living cells, and is performed under strict sterile and/or aseptic conditions. 3D bioprinting builds complex, cell-laden structures that can replicate tissue characteristics, with applications in regenerative medicine, drug testing, and tissue engineering. By layering bio-inks containing living cells, bioprinting has the potential to create tissue-like structures that could support cell growth, differentiation, and eventually integration into humans and other biological systems.

One of the primary challenges in 3D bioprinting is maintaining the viability and functionality of the printed cell-biomaterial constructs/structures, which are highly sensitive to environmental conditions. Cells and hydrogels, which make up the bio-ink, require a precise and stable hydration level to survive and function properly.

Dehydration during or after the bioprinting process can lead to cellular damage, reduced cell viability, compromised mechanical properties of the printed structure, and provide poor performance in relation to the intended purpose of the 3D structure. Even slight dehydration can cause shrinkage, warping, or structural weaknesses in the printed biomaterial, hindering its intended biological and mechanical performance.

The dehydration problems associated with 3D bioprinting prevents operators/users from bioprinting microstructures directly onto suitable surfaces without using additional solutions or moulds, which can provide further negative impacts.

Current methods to address dehydration often involve manual spraying, covering, or short-term enclosures, but these methods are limited in effectiveness, especially for longer-term preservation. Without an automated or consistent hydration system, printed biomaterials are at high risk of dehydration, especially in laboratory environments where humidity levels fluctuate, and in biosafety cabinets in which the relative humidity is around 30-38% or below. Consequently, there is a need for a dedicated system that can continuously and reliably keep 3D printed biomaterials hydrated.

Another problem associated with 3D bioprinting is the absence of a suitable surface for printing thereon, and a controlled environment to enable different bioprinting methods to be used. Such a surface and controlled environment is required for complex structures to be 3D bioprinted using robotic arms in a sterile manner over longer time periods.

Not all surfaces are amenable to 3D bioprinting. Surfaces must be hydrophobic to prevent the 3D bioprinted structures from collapsing upon printing or removal from the surface.

As the complexities and functional properties of the intended 3D structures increase, additional components are required to act simultaneously within a controlled environment. This most often requires different bioprinting technologies. As the end product holds live cells and may be used for medical applications, it is essential that every component that comes into contact with the bioprinting process needs to be sterile.

Current equipment does not have the capabilities or features to accommodate the above needs in a single system. In particular there is an unmet need for a single system which enables 3D bioprinting in a regulated and sufficiently humid environment.

The present invention was devised with the foregoing in mind.

### Summary of Invention

According to a first aspect of the invention, there is provided a device for generating liquid droplets.

The device may comprise a body configured to hold a liquid. The body may be configured to hold the liquid from which the liquid droplets are formed.

An inner surface of the body may be formed of, or comprise, one or more of: PFTE, polycarbonate, PFA and ABS.

The device may comprise one or more piezoelectric actuators. The piezoelectric actuators may be disposed within the body. The one or more piezoelectric actuators may be configured to oscillate at an ultrasonic frequency to atomise the liquid within the body to generate a plurality of droplets.

The use of piezoelectric actuators may enable the generation of liquid droplets which are sufficiently small to avoid damaging the structures which are to be hydrated. The use of piezoelectric actuators may enable the generation of liquid droplets with sufficient kinetic energy to diffuse over a sufficient distance within a sufficient timescale.

The device may comprise one or more piezoelectric sensors. The piezoelectric sensors may be disposed on an underside of the body. The piezoelectric sensors may be configured to generate an electrical signal when a force is applied upon them causing deformation. The piezoelectric sensors may be configured to generate an electrical signal when an airflow is directed upon them. The electrical signal may be configured to activate the piezoelectric actuators.

The piezoelectric actuators and piezoelectric sensors may be combined as one or more piezoelectric transducers. Each piezoelectric transducer may comprise at least one piezoelectric sensor and at least one piezoelectric actuator.

The use of piezoelectric sensors enables energy to be harvested from airflow and used to drive oscillation of the piezoelectric actuators. Bioprinting often occurs within a biosafety cabinet, or Laminar airflow cabinet, and so there is often high-speed airflow already present which can be utilised.

The device may be configured to generate droplets with a diameter between 0.5µm to 1.5µm.

The piezoelectric actuators may be configured to oscillate at a frequency between 0.1MHz and 3MHz. The piezoelectric actuators may be configured to oscillate at a frequency between 0.3MHz and 2.7MHz. The piezoelectric actuators may be configured to oscillate at a frequency between 0.8MHz and 2.4MHz.

The device may comprise a thermally insulating jacket. The thermally insulating jacket may at least partially surround the body. The thermally insulating jacket may be formed of, or comprise, ABS. The thermally insulating jacket may comprise one or more heating elements, such as infra-red heating elements. The thermally insulating jacket may comprise one or more cooling elements.

The use of a thermally insulating jacket, optionally with heating and/or cooling elements, may ensure that liquid within the body is maintained at an optimal temperature for droplet production.

The device may comprise a lid disposed at an end of the body. The lid may comprise an outlet aperture configured to receive an outlet tubing. The lid may be substantially dome shaped. Having a substantially dome-shaped lid, rather than a flat lid, may encourage droplets towards the outlet aperture.

The device may comprise a housing. The housing may comprise a base. The housing may comprise an enclosure. The enclosure may be configured to rest on the base. The body may be disposed within the enclosure.

The enclosure may provide further thermal insulation to the liquid within the body and the droplets generated. The base may redirect surrounding airflow onto the piezoelectric sensors.

The base of the housing may comprise one or more side vents to enable airflow therethrough. The enclosure may comprise a bottom surface comprising an opening aligned with a bottom surface of the body.

The vents may comprise a plurality of struts. The struts may comprise left-inclined struts and right-inclined struts.

The left-inclined struts may be angled relative to the horizontal bottom of the base at an angle equal to, or greater than, 90°. The left-inclined struts may be angled relative to the horizontal bottom of the base at an angle between 100-130°. The left-inclined struts may be angled relative to the horizontal bottom of the base at an angle between 110-120°. The left-inclined struts may be angled relative to the horizontal bottom of the base at an angle of 114°.

The right-inclined struts may be angled relative to the horizontal bottom of the base at an angle between 0 and 90°. The right-inclined struts may be angled relative to the horizontal bottom of the base at an angle between 40 and 60°. The right-inclined struts may be angled relative to the horizontal bottom of the base at 50°.

Each of the left-inclined struts may be parallel with each other. Each of the left-inclined struts may be separated 23 mm apart. Each of the left-inclined struts may be separated between 15-30mm apart. Each of the left-inclined struts may be separated between 20-25mm apart.

Each of the rights-inclined struts may be parallel with each other. Each of the right-inclined struts may be separated 23 mm apart. Each of the right-inclined struts may be separated between 15-30mm apart. Each of the right-inclined struts may be separated between 20-25mm apart.

The left-inclined struts may have a diamond-shaped (i.e., rhombus) cross-section. The left-inclined struts may have a diamond-shaped (i.e., rhombus) cross-section with two small angles of 30° and two large angles of 150°. The left-inclined struts may be arranged such that a vertex with an angle of 30° directly faces outwards to receive the incoming airflow.

The right-inclined struts may have a diamond-shaped (i.e., rhombus) cross-section. The right-inclined struts may have a diamond-shaped (i.e., rhombus) cross-section with two small angles of 60° and two large angles of 120°. The right-inclined struts may be arranged such that a vertex with an angle of 60° directly faces outwards to receive the incoming airflow.

The height of the vents may be between 40-70mm. The height of the vents may be between 50-60mm. In some examples, the height of the vents may be 55mm.

According to a second aspect of the invention, there is provided a hydration chamber.

The hydration chamber may comprise a chamber lid configured to fit over a printing plate. The chamber lid may comprise an inlet. The inlet may be configured to enable passage of liquid droplets therethrough. The chamber lid may comprise sidewalls.

The hydration chamber may comprise the printing plate. The printing plate may be formed of, or comprise, one or more of: PFTE, PFA and polycarbonate.

The hydration chamber may be for use with an external printing plate of a 3D bioprinter.

The chamber lid may be configured to enclose a volume of air surrounding a printing plate. The hydration chamber may enable the volume of air surrounding the printing plate (which may be part of the hydration chamber) to be monitored and controlled, and to be protected from ingress of contaminants.

The chamber lid may comprise an internal surface configured to face the plate. The internal surface may be angled between 10-35° relative to the plate, when in use. Having a chamber lid with an angled inner surface may ensure that liquid droplets formed on an inner surface of the chamber lid move under the influence of gravity towards a side of the chamber lid, rather than dropping onto a structure formed on the plate.

The hydration chamber may comprise a plate jacket. The plate jacket may be thermally insulating. The plate jacket may be configured to at least partially surround the printing plate. The plate jacket may be configured to fully cover at least one surface of the printing plate.

The plate jacket may be formed of, or comprise, polycarbonate, PFA and/or Acrylonitrile butadiene styrene (ABS).

The hydration chamber may comprise a chamber lid jacket. The chamber lid jacket may be configured to at least partially surround the chamber lid. The chamber lid jacket may be thermally insulating.

The chamber lid jacket may be formed of, or comprise, polycarbonate, PFA and/or ABS.

The chamber lid jacket may be formed of a plurality of modular regions. The modular regions may be removable and replaceable. Having a modular chamber lid jacket may enable the chamber lid jacket to be used with a plurality of different chamber lids.

The plate jacket may comprise one or more heating and/or cooling elements configured to increase or decrease the temperature of the plate. The chamber lid jacket may comprise one or more heating and/or cooling elements configured to increase or decrease the temperature of the chamber lid and the air enclosed within the chamber. Having heating elements to increase the temperature of the chamber lid may prevent or reduce condensation forming on the chamber lid.

The hydration chamber may comprise one or more probes. The one or more probes may be configured to measure one or more environmental parameters in the space between the plate and the chamber lid. The one or more probes may be configured to measure one or more environmental parameters in the space enclosed by the hydration chamber. The one or more probes may comprise a hydrostat. The one or more probes may comprise a hygrostat The one or more probes may comprise a temperature sensor (i.e., a thermometer). The plate jacket or chamber lid jacket may comprise one or more openings through which the probes can pass.

The chamber lid may comprise one or more openings protected by sleeves. The sleeves may provide access to the plate through the lid. The sleeves may provide access to the plate through the lid whilst preventing ingress of contaminants. The sleeves may be made of, or comprise, silicone and/or polypropylene.

The sleeves may enable robotic arms and/or components of a bioprinter to access the printing plate whilst maintaining the controlled environment and without allowing ingress of contaminants.

The system may comprise a wall extension. The wall extension may be disposed between the plate and the chamber lid. The wall extension may be formed of, or comprise, polycarbonate. The wall extension may be transparent.

According to a third aspect of the invention, there is provided a system for maintaining hydration of a biostructure. The system for maintaining hydration may comprise the device of the first aspect of the invention. The system for maintaining hydration may comprise the hydration chamber of the second aspect of the invention.

The system may comprise a tubing configured to convey liquid droplets generated by the device of the first aspect into the hydration chamber of the second aspect through the inlet.

Features which are described in the context of separate aspects and embodiments of the present invention may be used together and/or be interchangeable wherever possible. Similarly, where features are described in the context of a single embodiment for brevity, those features may also be provided separately or in any sub-combination. Features described in connection with the device of the first aspect may have corresponding features definable with respect to the hydration chamber of the second aspect, or the system of the third aspect, and vice versa, and those embodiments are specifically envisaged.

### Brief description of the drawings

The invention will now be described, by way of example only, with reference to the accompanying drawings, in which:
**Figure 1** shows a schematic of a device for generating liquid droplets;
**Figures 2(a) and 2(b)** show schematic side and bottom views of a device for generating liquid droplets;
**Figures 3(a) and 3(b)** respectively show enlarged perspective views of example piezoelectric sensors and piezoelectric transducers.
**Figure 4(a)** shows a perspective view of a device for generating liquid droplets disposed within a housing, and **Figure 4(b)** shows a perspective view of a lid of the housing;
**Figure 5** shows a perspective view of an enclosure of a housing;
**Figure 6** shows a perspective view of a base of a housing;
**Figure 7** shows a schematic of a device for generating liquid droplets connected to a hydration chamber;
**Figure 8** shows a schematic of a hydration chamber;
**Figure 9** shows a schematic of a hydration chamber comprising a lid;
**Figure 10** shows s schematic of a hydration chamber comprising an angled thermal jacket;
**Figures 11(a) and 11(b)** respectively show perspective and side views of a lid for a hydration chamber;
**Figures 12(a) and 12(b)** respectively show perspective and side views of a lid jacket for a lid of a hydration chamber;
**Figures 13(a) and 13(b)** show perspective views of lids for a hydration chamber comprising sleeves;
**Figures 14(a) and 14(b)** show perspective views of lids for a hydration chamber;
**Figures 15(a) and 15(b)** show perspective views of modular jackets for a lid for a hydration chamber; and
**Figure 16** shows a perspective view of a wall extension for a plate of a hydration chamber.

### Detailed description

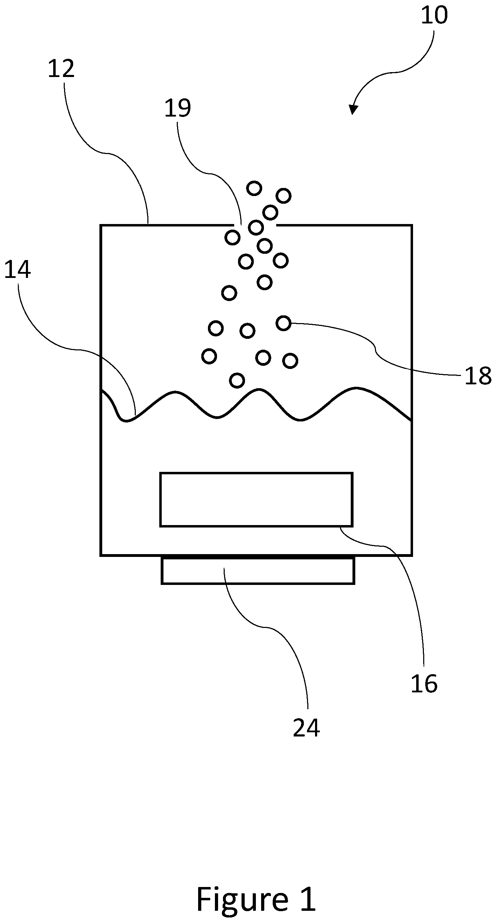

Figure 1 shows a schematic of a device 10. The device 10 is configured to generate liquid droplets.

The device 10 comprises a body 12. The body 12 is configured to hold a liquid 14, such as water.

The device 10 comprises a piezoelectric actuator 16. The piezoelectric actuator 16 is disposed within the body 12. Although a single piezoelectric actuator 16 is shown in Figure 1, in other examples there can be a plurality of piezoelectric actuators 16.

The piezoelectric actuator 16 is disposed near to the bottom of the body 12 such that, when a liquid 14 is held within the body 12, the piezoelectric actuator 16 is fully submerged in the liquid 14. In some examples, the piezoelectric actuator 16 is disposed at the bottom of the body 12.

The piezoelectric actuator 16 is configured to oscillate. The piezoelectric actuator 16 is configured to oscillate at an ultrasonic frequency. In some examples, the piezoelectric actuator 16 is configured to oscillate at a frequency between 0.1MHz and 3MHz. In some examples, the piezoelectric actuator 16 is configured to oscillate at a frequency between 0.3MHz and 2.7MHz. In some examples, the piezoelectric actuator 16 is configured to oscillate at a frequency between 0.8MHz and 2.4MHz.

The oscillation of the piezoelectric actuator 16 causes atomisation of the liquid 14 within the body 12 to generate a plurality of liquid droplets 18.

The atomisation of the liquid 14 occurs due to the superposition of two effects which can be explained under the capillary wave theory and the cavitation principle. The piezoelectric actuator 16 oscillates with a frequency in the ultrasonic range, and the cyclic change of amplitude of the piezoelectric actuator 16 generates a momentary vacuum in the nearby liquid body 14 due to the inertia of the liquid 14. Once the oscillation speed increases, a momentary vacuum and strong compression force occurs at a speed where the water particles can no longer follow the oscillating surface, resulting in an explosive formation of minute air bubbles. This is known as cavitation.

At the cavitation bubble sites, bubble implosion occurs to generate liquid droplets 18 which break the surface tension and dissipate to the air in vapor form, and subsequently get absorbed into an air stream. In addition, capillary waves are generated in the liquid 14. Minute water aerosols are also emitted to the air at the crests of the Rayleigh surface waves that are regularly generated by the oscillating piezoelectric actuator 16.

In some examples, the liquid droplets generated have an average diameter between 0.1µm to 5µm. In some examples, the liquid droplets generated have an average diameter between 0.5µm to 5µm. In some examples, the liquid droplets generated have an average diameter between 0.5µm to 1.5µm.

The device 10 comprises one or more piezoelectric sensors 24 which are configured to convert airflow induced vibrations into an electrical signal. This electrical signal is used to power and control the piezoelectric actuators 16. The piezoelectric sensors 24 enable energy to be harnessed from surrounding airflow and used to drive oscillation of the piezoelectric actuators 16.

The piezoelectric sensors 24 are connected to the body 12. The sensors can be connected to an underside of the body 12 and exposed to airflow onto the underside of the body.

In some examples, the piezoelectric actuators 16 and piezoelectric sensors 24 are part of components referred to as piezoelectric transducers. A piezoelectric transducer may refer to the pairing of at least one piezoelectric actuator 16 with at least one piezoelectric sensor 24.

The device 10 can comprise one or more piezoelectric transducers. The piezoelectric transducers can be configured such that the piezoelectric sensors 24 use pressure induced by an incoming airflow to produce an electrical signal which is used to actuate the piezoelectric actuators 16. The electrical signal generated by the piezoelectric sensors 24 can be proportional to the speed of the incoming airflow.

The piezoelectric transducers can be formed of, or comprise, ceramics. The piezoelectric transducers can be formed of, or comprise, composite materials. In some examples, the piezoelectric transducers are formed of, or comprise, Lead Zirconate Titanate.

The device 10 comprises an outlet 19. The outlet 19 enables the liquid droplets 18 to exit the body 12. In the example of Figure 1, the outlet 19 is an aperture in the top of the body 12.

In use, the device 10 is configured such that the liquid droplets 18 generated from the liquid 14 by the piezoelectric actuators 16 are conveyed towards biological material in order to rehydrate, or maintain hydration, of the biological material. In some examples, the device 10 is connected to a hydration chamber and conveys liquid droplets into the chamber. Some such chambers are discussed in greater detail later in this specification but the hydration device can be used in conjunction with any hydration chamber and/or plate.

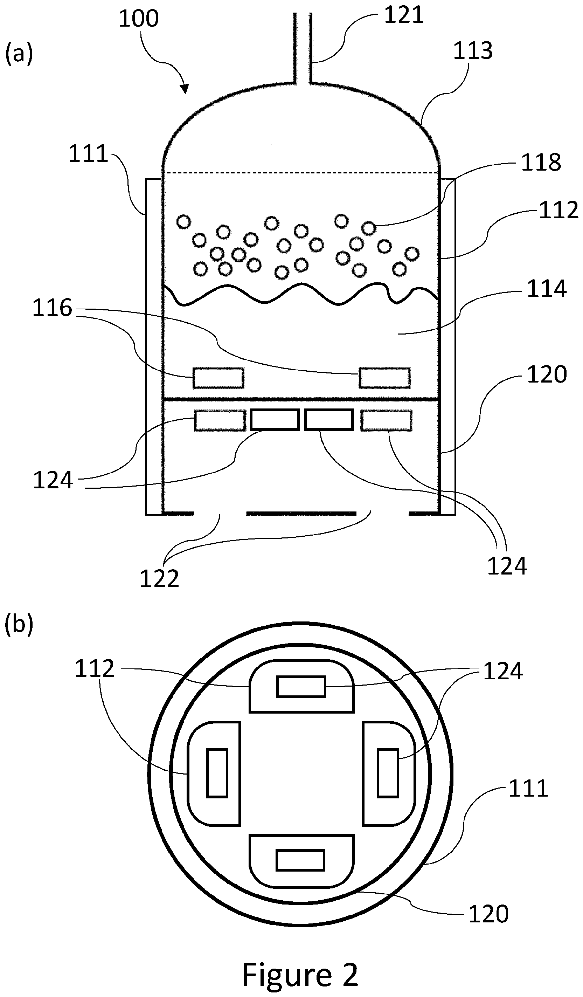

Figure 2(a) shows a cross-sectional side view of a device 100 for generating liquid droplets. Figure 2(b) shows a bottom view of the device 100 of Figure 2(a). The device 100 is a specific example of the generic device 10 shown in Figure 1.

The device 100 comprises a cylindrical body 112. The body 112 is configured to hold a liquid 114, as discussed in relation to the device 10 of Figure 1. In some examples, the inner surface of the body 112 is made of, or comprises, PTFE. In other examples, the inner surface of the body is made of, or comprises, ABS, PFA and/or polycarbonate. Although the body 112 is cylindrical in this example, the skilled person will recognise that the body 112 can have alternative shapes in other examples.

Four piezoelectric actuators 116 are disposed within the body 112 and, in the example shown, are submerged within the liquid 114. In other examples, any number of piezoelectric actuators 116 can be used.

The device 100 comprises a thermally insulating jacket 111 surrounding the body 112. In some examples, the thermally insulating jacket is formed of, or comprises, ABS. In other examples, the jacket can be omitted entirely.

In some examples, the thermally insulating jacket 111 comprises one or more heating elements, such as infra-red heating elements. The heating elements and/or the thermal insulation provided by the jacket 111 can be used to maintain the liquid 114 within the body 112 at a desired temperature or within a desired temperature range. For example, the liquid 114 within the body 112 can be held between 30-70°C or, more specifically, 33.3-68.8°C. Temperatures within this range enhance the cavitation bubble implosion by reducing the surface tension of the liquid, thereby enhancing the droplet formation.

The device 100 comprises a lid 113. The lid 113 is hemispherical (i.e., dome shaped) in the present example but, in other examples, the lid can have any other shape. The dome-shape of the lid 113 conveys the droplets 118 towards an aperture positioned at the centre of the lid 113.

A tube 121 extends from the aperture at the centre of the lid 113. The tube 121 is configured to convey water droplets 118 towards a desired destination, such as a printing plate of a 3D printer. In other examples the aperture, and tube 121 extending therefrom, can be off-centre.

In some examples, the tube 121 is integral with the lid 113. In other examples, the tube 121 is connectable to the lid 113, for example, via a screw fitting, bayonet fitting, or any other suitable means for connecting the tube 121 to the lid 113.

The device comprises an airflow portion 120. The airflow portion 120 is connected to the body 112, on an opposite side to the lid 113 and tube 121. The airflow portion 120 is positioned such that, when the device is in use, the airflow portion 120 is beneath the body 112.

The airflow portion 120 is a substantially open container. The airflow portion 120 comprises openings 122 through which air can flow into the airflow portion 120. The airflow portion 120 is empty except for sensors 124. The sensors 124 are piezoelectric sensors. In the example of Figure 2(a) there are four sensors 124 but, in other examples, there can be one sensor, or any plurality of sensors. In the example of Figure 2(a), there are four openings 122 but, in other examples, there can be one opening, or any plurality of openings. In other examples, the airflow portion 120 and the sensors 124 can be omitted entirely.

Figure 2(b) shows the underside (i.e., the bottom) of the airflow portion 120. The airflow portion 120 comprise four openings 112 arranged circumferentially. Sensors 124 are visible through each of the openings 112.

In use, the device 100 can be disposed within a biosafety cabinet, or laminar airflow (LAF) cabinet. Such cabinets are configured to provide an airflow, for example via one or more fans, to prevent contaminants passing in or out. The device 100 is configured to exploit this airflow via the sensors 124. The airflow is directed via the openings 122 into the airflow portion 120 and onto the piezoelectric sensors 124. The piezoelectric sensors 124 deform as a result of the airflow, thus producing an electrical signal via the piezoelectric effect. This electrical signal is used in turn to power/control the piezoelectric actuators 116. By harnessing energy from the airflow present in the surrounding environment, the input from a battery or other power source required by the device 100 is reduced or removed.

In other examples, the device 100 can comprise, or be utilised in conjunction with, one or more fans to increase the airflow into the airflow portion and/or onto the sensors 124. A DC power source, such as a battery, can be used to power said fans.

In other examples, the device 100 can comprise one or more power sources to provide power to the piezoelectric actuators 116. The power source(s) can be used instead of, or in addition to, the piezoelectric sensors 124 to provide power to the piezoelectric actuators 116.

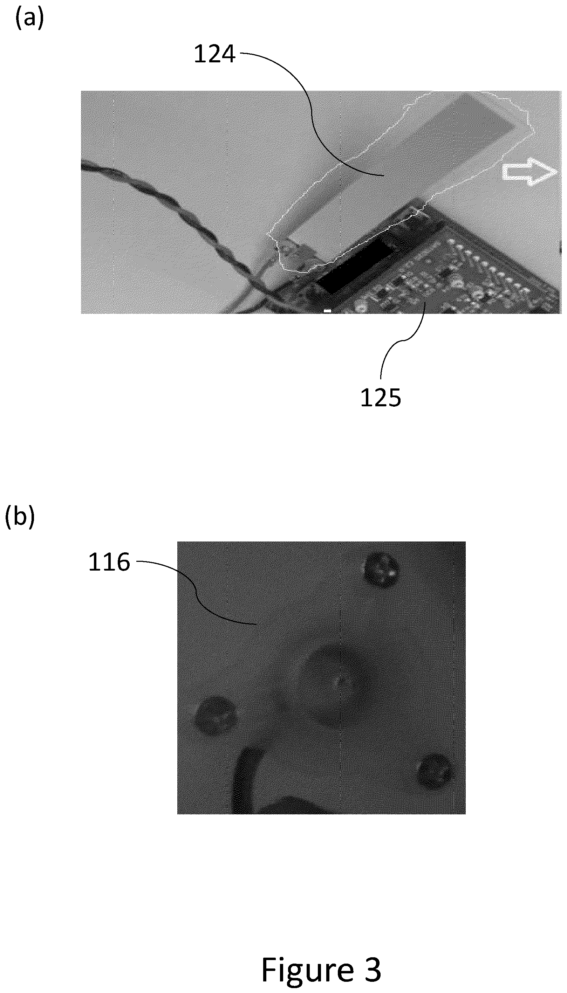

Figures 3(a) and 3(b) respectively show enlarged perspective views of example piezoelectric sensors 124 and piezoelectric transducers 116.

Figure 3(a) shows a piezoelectric sensor 124 used in some examples. The sensor 124 is configured to generate an electric signal in response to deformation (i.e., due to airflow upon it). In the example shown, the sensor 124 is connected to a processor 125. In some examples, the processor 125 is a microcontroller. The electric signal generated by the sensor 124 is sent, via the processor 125, to the piezoelectric transducers 116. In some examples, the processor 125 amplifies the signal received from the sensor 124. In some examples, the processor/microcontroller 125 controls the frequency at which the piezoelectric actuator 116 oscillates. For example, the processor 125 can be configured such that the actuator 116 oscillates at a preset frequency, or within a preset frequency range. In other examples, the processor 125 can be omitted entirely.

Figure 3(b) shows a piezoelectric actuator 116 within a protective casing. The actuator 116 is submerged within a liquid 114 (see Figure 2), and the casing is configured to isolate the electrical connections from said liquid 114. The casing is configured to expose only an actuating portion (i.e., an actuating plate) of the piezoelectric actuator 116. In the example shown, the casing is fixed to the bottom of the body 112 via screws.

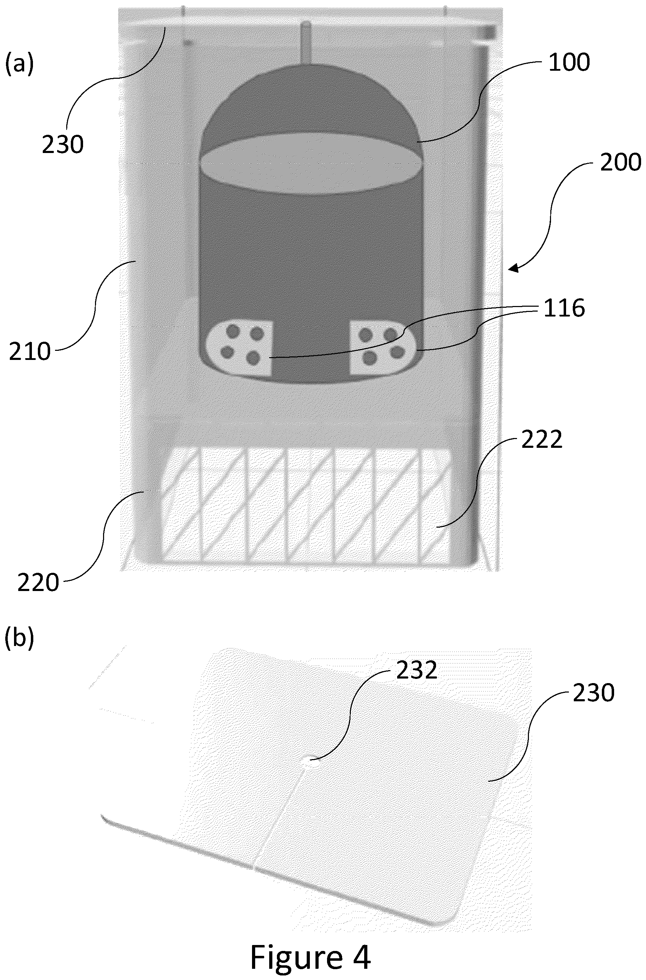

Figure 4(a) shows a perspective view of a device 200. The device 200 comprises the device 100 together with a housing. The housing comprises a base 220 and an enclosure 210.

In some examples, the enclosure 210 is formed of, or comprises, ABS and PTFE and/or PFA. An inner surface of the enclosure 210 is formed of, or comprises, PTFE and/or PFA. An outer surface of the enclosure 210 is formed of, or comprises, ABS. The base 220 can be formed similarly to the enclosure 210.

The enclosure 210 is configured to rest on top of the base 220. The device 100 is disposed within the enclosure 210 and rests on top of the base 220.

The housing comprises a lid 230. Figure 4(b) shows a perspective view of the lid 230. The lid 230 is configured to rest on top of the enclosure 210. The lid 230 comprises a central aperture 232. In use, the tube 121 of the device 100 extends through the aperture 232 of the lid 230.

The lid 230 comprises a slit extending from the central aperture 232 to an edge of the lid 230. The slit allows easy detachment and assembly, and enhanced ambidextrous operation, giving greater freedom of movement for the outlet tubing 121 connected to the hydration chamber.

In some examples, the lid 230 is formed of, or comprises, ABS, PFA and/or PTFE. In some examples, an inner surface of the lid 230 (i.e., the surface which faces device 100 when in use) is formed of, or comprises, PTFE and/or PFA. In some examples, the outer surface can be formed of, or comprise, ABS or PTFE or PFA.

The enclosure 210 and the lid 230 provide further thermal insulation to the device 100. In some examples, the enclosure 210 and/or the lid 230 comprise one or more heating/cooling elements, such as IR heating elements, or Peltier systems. The enclosure 210 and lid 230, together with any heating/cooling elements contained therein, can be used to maintain a surrounding temperature for the device 100.

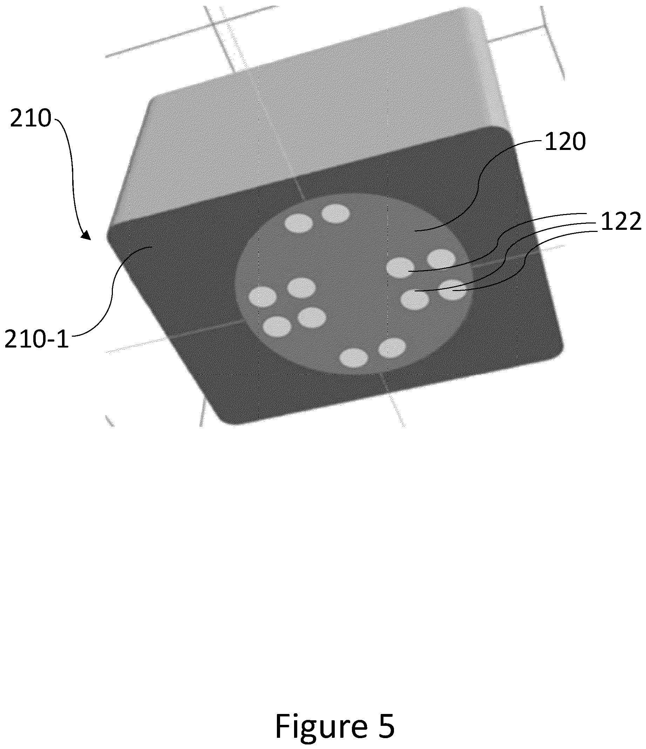

Figure 5 shows a perspective bottom view of the enclosure 210. The enclosure 210 is substantially rectangular with curved edges, defining a central space in which the device 100 can be disposed. In other examples, the enclosure 210 can have an alternative shape.

The enclosure comprises a bottom surface 210-1. The bottom surface 210-1 comprises an opening. In use, the device 100 is aligned with the opening such that the bottom of the air flow portion 120 of the device 100 covers the opening. As shown in Figure 4, positioning the airflow portion 120 over the opening of the bottom surface 210-1 exposes the openings 122 of the air flow portion 120. In Figure 5, ten circular openings 122 are shown which enable airflow into the air flow portion 120.

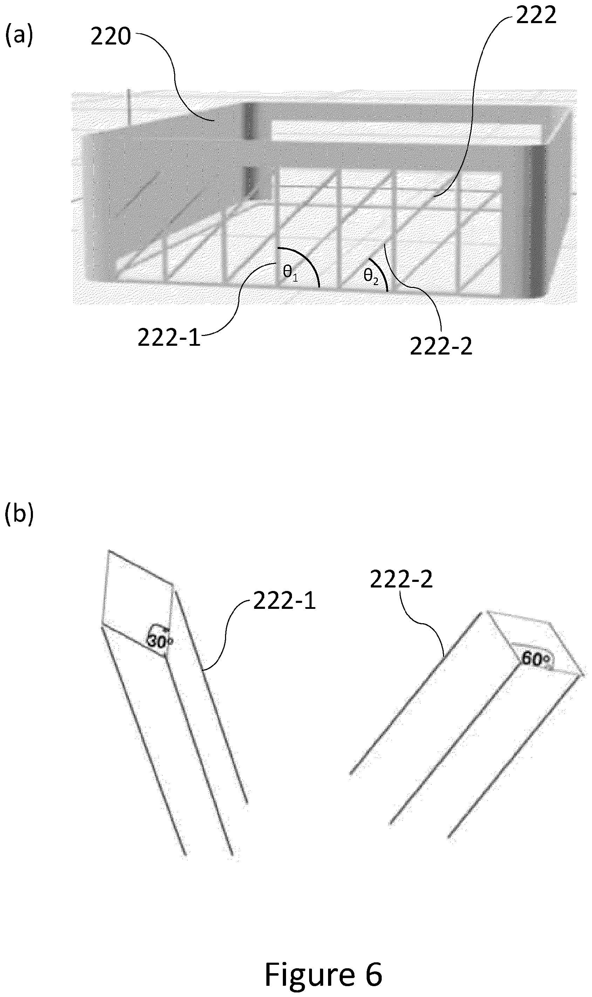

Figure 6(a) shows a perspective view of the base 220. The base 220 comprises side vents 222 which enable airflow therethrough.

The base 220 suspends the enclosure relative to a surface upon which the base 220 is disposed and enables airflow, via the vents 222, towards the underside 210-1 of the enclosure and the openings 122 of the device 100. As explained above, the airflow via the vents 222 is used to power and control the piezoelectric actuators.

In the example of Figure 6(a), the vents 222 are formed of a series of left-inclined struts (222-1) and right-inclined struts (222-2).

The left-inclined struts (222-1) are angled relative to the horizontal bottom of the base 220 at an angle θ₁. In the example of Figure 6(a), θ₁ is 114°. In other examples, θ₁ can be any angle equal to, or greater than, 90°. θ₁ in this example is being measured rotating counterclockwise from the horizontal.

The right-inclined struts (222-2) are angled relative to the horizontal bottom of the base 220 at an angle θ₂. In the example of Figure 6(a), θ₂ is 50°. In other examples, θ₂ can be any value between 0 and 90°. θ₂ in this example is being measured rotating counterclockwise from the horizontal.

The left-inclined struts (222-1) are parallel with each other and are separated 23 mm apart. In other examples, the distance between consecutive struts can be different. In some examples, the distance between consecutive struts is between 15-30mm. In some examples, the distance between consecutive struts is between 20-25mm.

The right-inclined struts (222-2) are parallel with each other and are separated 23 mm apart. In other examples, the distance between consecutive struts can be different. In some examples, the distance between consecutive struts is between 15-30mm. In some examples, the distance between consecutive struts is between 20-25mm.

As shown in Figure 6(b), the left-inclined struts (222-1) have a diamond-shaped (i.e., rhombus) cross-section with two small angles of 30° and two large angles of 150°. The left-inclined struts (222-1) are arranged such that a vertex with an angle of 30° directly faces outwards to receive the incoming airflow.

As shown in Figure 6(b), the right-inclined struts (222-2) have a diamond-shaped (i.e., rhombus) cross-section with two small angles of 60° and two large angles of 120°. The right-inclined struts (222-2) are arranged such that a vertex with an angle of 60° directly faces outwards to receive the incoming airflow.

In some examples, the height of the vents 222 is between 40-70mm. In some examples, the height of the vents 222 is between 50-60mm. In some examples, the height of the vents 222 is 55mm.

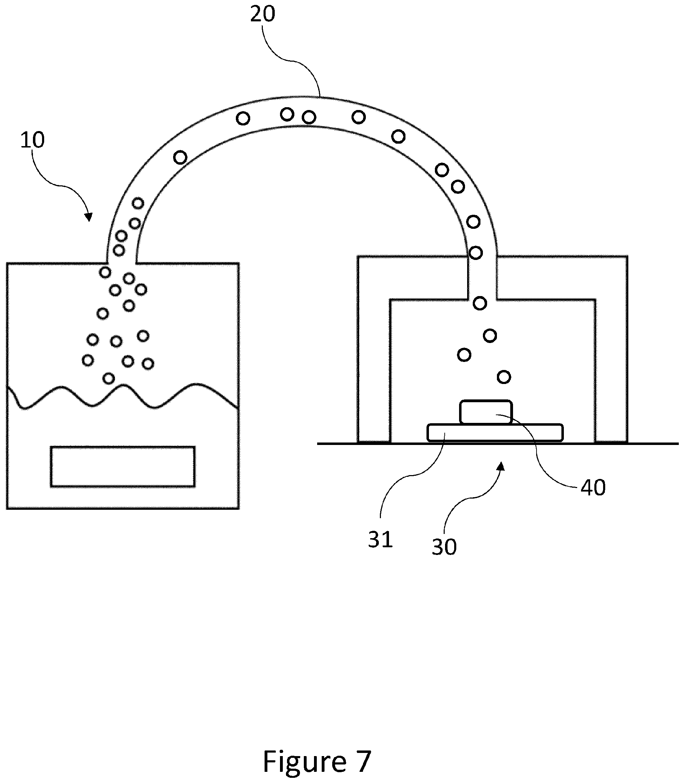

Figure 7 shows a schematic of the device 10 of Figure 1 in use with a hydration chamber 30. In other examples, the device 10 of Figure 1 or the device 100 of Figures 2-6 can be used with an existing plate of a 3D printer, rather than a dedicated hydration chamber. The device 10 of Figure 1 or the device 100 of Figures 2-6 can also be used in any other situation wherein liquid droplets are required.

A tubing 20 is connected to the outlet 19 of the device 10. The tubing 20 is configured to convey liquid droplets generated by the device 10 to the hydration chamber 30.

The hydration chamber 30 defines an environment in which a biostructure 40 can be printed and/or maintained following printing.

In some examples, the floor of the hydration chamber 30 itself could be used as a bioprinting surface on which the biostructure 40 is 3D-printed directly. That is to say, the hydration chamber 30 can comprise a plate 31 upon which a biostructure 40 is disposed. For example, the biostructure 40 could be biological material which has been 3D-printed onto the floor of the hydration chamber 30 directly. In other examples, the hydration chamber 30 could be used for multiple other purposes.

Alternatively, a separate plate 31 for supporting 3D bioprinting could be accommodated in the hydration chamber, rather than the plate 31 being part of the hydration chamber 30 itself. The plate 31 can be part of a 3D bioprinting device or can be a separate plate 31 onto which the structure 40 can be transferred. The plate 31 can be a temperature-controlled plate.

The hydration chamber 30 can reside on the temperature-controlled (i.e., heated, or chilled) plate of most existing bioprinters.

The structure 40 requires sufficient hydration to avoid permanent damage. The structure 40 is hydrated via the liquid droplets generated by the device 10.

When the hydration chamber is on the bioprinter many options exist. It can be used with the vertical spacer or even without the vertical spacer. Vertical spacer gives more volume and space for the chamber and visibility to observe if one needs. It can be with any specific lid thus mentioned without the thermal jackets or with the lid and the thermal jacket based on user preference.

Example hydration chambers 30 are discussed in greater detail below.

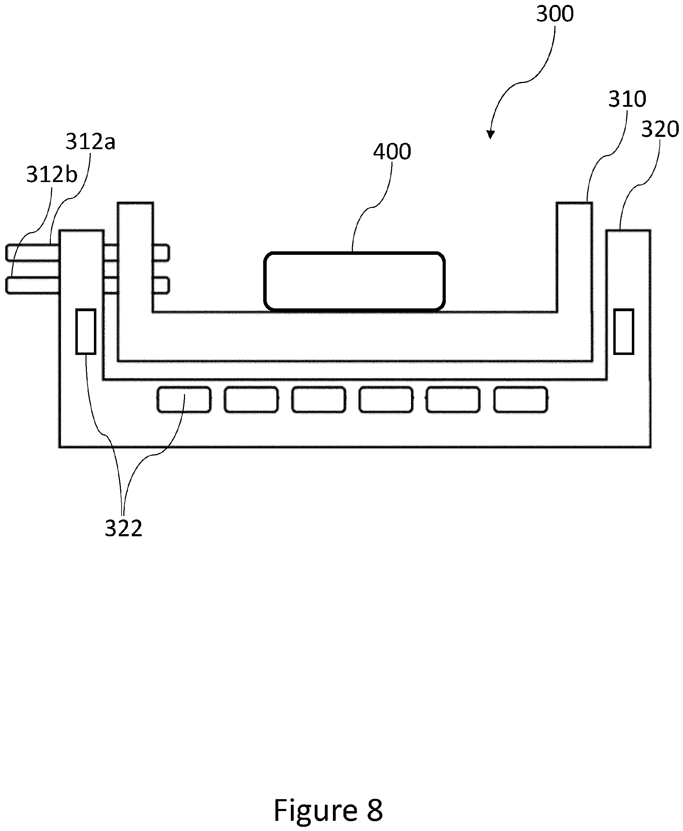

Figure 8 shows a schematic diagram of a hydration chamber 300.

The hydration chamber 300 comprises a plate 310. The plate 310 comprises a flat portion with sidewalls.

The hydration chamber 300 comprises a jacket 320. The jacket 320 surrounds the plate 310. The jacket 320 is thermally insulating and is configured to prevent heat transfer between the plate 310 and the external environment. The jacket 320 can also provide cooling and/or heating functions, as required, to the structure 400.

The jacket 320 can be used, for example, when the hydration chamber 300 is used with an external bioprinter's printing plate, without utilizing the heat/cooling function of the bioprinter's printing plate. In other examples, if a plate of a bioprinter is used which comprises its own heating/cooling functions, the hydration chamber 300 can be used without a jacket 320.

In some examples, the plate 310 is formed, or comprises, PTFE and/or PFA.

In some examples, the plate 310 has a thickness of less than 5mm. In some examples, the plate 310 has a thickness between 1-3.5mm.

In some examples an inner surface of the jacket 320 is formed of, or comprises, PTFE or PFA. In some examples, an outer surface of the jacket 320 is formed of, or comprises, polycarbonate, or ABS.

The plate 310 comprises openings in the sidewalls configured to enable probes 312a, b to pass therethrough. The jacket 320 comprises openings which correspond to the openings of the sidewall of the plate 310 such that, when the jacket 320 surrounds the plate 310, probes 312a, b can pass through the openings of the jacket 320 and the plate 310 simultaneously.

The hydration chamber 300 comprises a thermostat probe 312a and a hygrostat probe 312b. The thermostat probe 312a is configured to measure the temperature of, and/or the region surrounding, the plate 310. The hygrostat probe 312b is configured to measure the relative humidity in the region surrounding the plate 310. In other examples, additional and/or alternative probes can be used.

The use of probes 312a, b enables monitoring of the conditions in which the biostructure 400 is placed.

In the example of Figure 8, there are two probes 312a, b. In other examples, there can be one probe, no probes, or any plurality of probes, together with the associated number of openings in the plate 310 and jacket 320.

The jacket 320 comprises a plurality of heating/cooling elements 322. The heating/cooling elements 322 are configured to transfer heat to/away from the plate 310 so as to increase/decrease the temperature of the plate 310. In some examples, the heating/cooling elements 322 can be activated in response to the thermostat probe 312a detecting that the temperature is too low/high for the biostructure 400.

Although not shown in the figures, the probes 312a, b can be connected to a processor. The processor can be configured to transfer data from the probes to an external device, analyse data from the probes, or send data from the probes to be displayed to a user.

In some examples, the heating elements 322 of the jacket 320 are arranged to provide a plurality of heating regions such that different regions of the plate 310 can be maintained at different temperatures, and such that specific temperature gradients can be formed across the plate 310. The heating elements 322 can be individually controllable.

The heating elements 322 are shown in Figure 8 as being enclosed within the jacket 320. In other examples, the heating elements 322 can be partially exposed, but only if the heating elements are able to be sterilized. In some examples, the heating elements 322 are infrared heating elements.

In some examples, a Peltier cooling system could be integrated within the jacket 320 to provide cooling and/or heating to the plate 310.

In some examples, if the hygrostat probe 312b measures that the humidity is too low for the biostructure 400, a hydration device (such as hydration device 10 of Figure 1 or hydration device 100 of Figure 2) can be switched on to provide more droplets, or the amount of droplets coming from the hydration device can be increased.

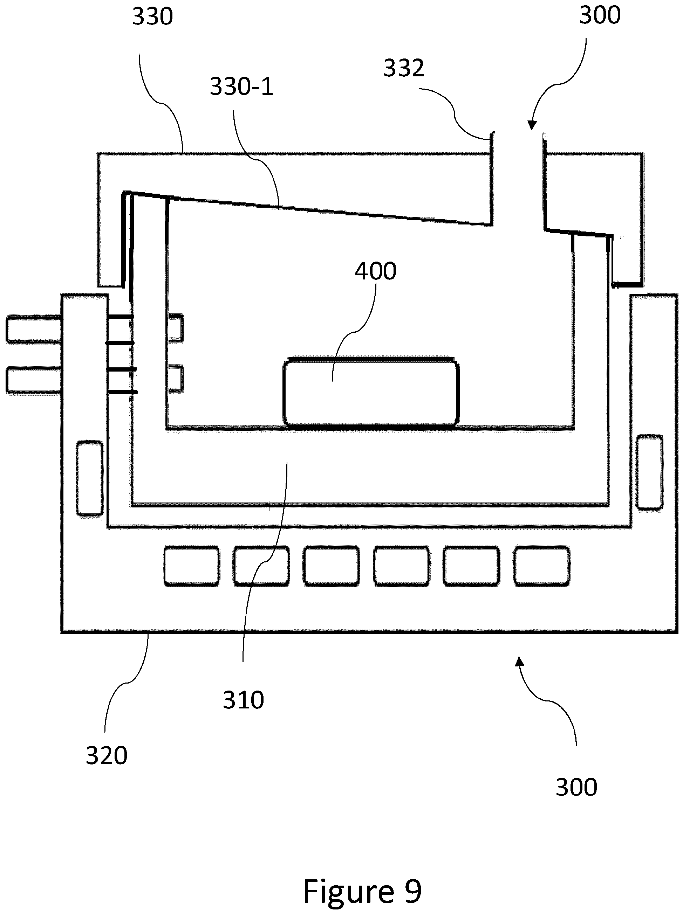

Figure 9 shows the hydration chamber 300 with a lid 330. The lid 330 is configured to substantially enclose the volume surrounding the plate 310. The lid 330 is configured to substantially enclose the volume surrounding the biostructure 400.

In the example of Figure 9 the lid 330 is fitted on top of the plate 310 such that the lid 330 rests on the sidewalls of the plate 310, and the sidewalls of the lid 330 surround sidewalls of the plate 310.

In other examples, the lid 330 may enclose the volume surrounding the plate 310 without resting on the sidewalls of the plate 310. For example, sidewalls of the lid 330 can rest on the base of the plate directly to enclose the portion of the plate upon which the structure 400 is present, of the lid 330 can cover the entire plate 310 and rest on an external surface which supports the plate 310.

The lid 330 comprises an opening 332. The opening 332 is configured to enable liquid droplets to pass therethrough. In some examples, a tubing is integral with the lid 330 which is configured to convey liquid droplets through the opening 332 of the lid 330. In other examples, the tubing is separate from, and connectable to, the lid 330.

Although the opening 332 is shown in Figure 9 to be disposed centrally along the length of the lid 330, in other examples it can be offset from the centre. Similarly, the opening 332 can be offset centrally across the width of the lid 330 is some examples.

The lid 330 comprises an internal surface 330-1. The internal surface 330-1 of the lid 330 is configured, in use, to face the plate 310. The lid 330 is configured such that, when it is positioned over the plate 310, the internal surface 330-1 is sloped/angled relative to the horizontal.

In some examples, the internal surface is angled between 0-45° relative to the plate 310 and/or the horizontal. In some examples, the internal surface 330-1 is angled between 10-35° relative to the plate 310 and/or the horizontal. In some examples, the internal surface is angled between 20-25° relative to the plate 310 and/or the horizontal. In some examples, the internal surface is angled at 23° relative to the plate 310 and/or the horizontal.

The slope of the internal surface 330-1 of the lid 330 encourages any liquid droplets disposed/formed on the lid 330 to run towards the side of the lid 330. This prevents liquid droplets from falling from the centre of the lid 330 onto a biostructure 400 disposed at or near the centre of the plate 310.

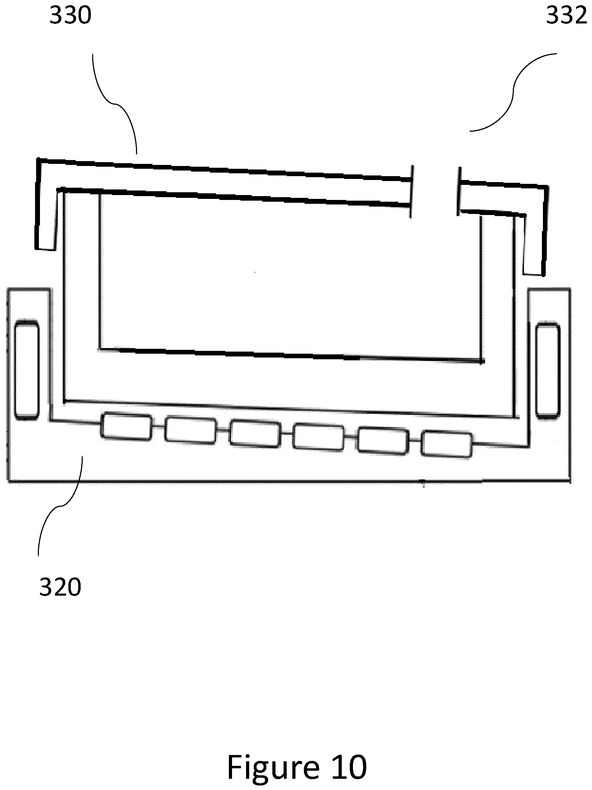

Alternatively, as shown in Figure 10, to acquire the same benefits, the interior surface of the heating/cooling jacket 320 can be angled relative to the horizontal, such that liquid droplets formed on the lid run towards a side of the lid. In some examples, the internal surface is angled between 0-45° relative to the plate 310 and/or the horizontal. In some examples, the internal surface is angled between 10-35° relative to the plate 310 and/or the horizontal. In some examples, the internal surface is angled between 20-25° relative to the plate 310 and/or the horizontal. In some examples, the internal surface is angled at 23° relative to the plate 310 and/or the horizontal.

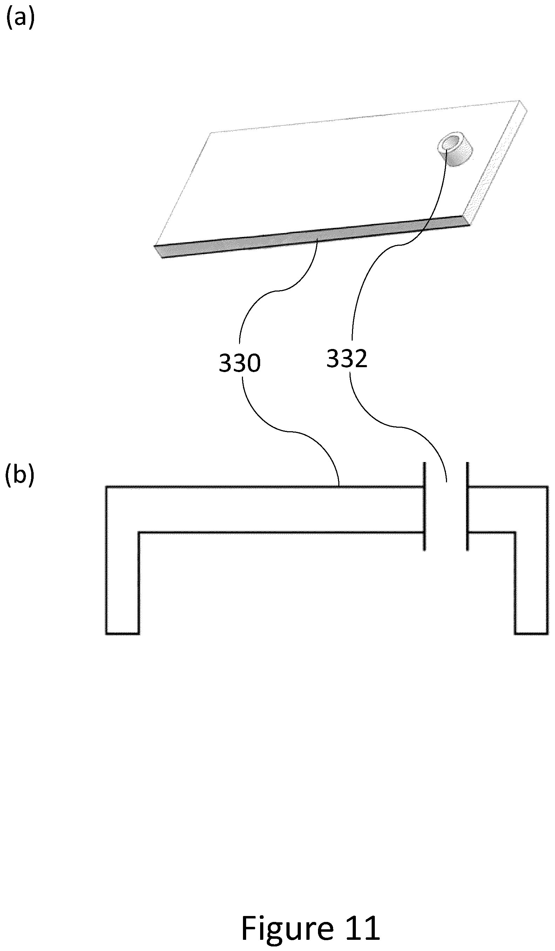

Figures 11(a) and 11(b) respectively show perspective and schematic side views of a lid 330 for a hydration chamber. The lid 330 is shown in a simplified form such that the angled inner surface is not visible.

In some examples, the lid 330 is made of, or comprises, polytetrafluoroethylene (PTFE). In other examples, the lid 330 is made of, or comprises, polycarbonate and/or Perfluoro alkoxy alkanes (PFA).

The opening 332 of the lid 330 is shown to be offset from the centre along the length of the lid 330.

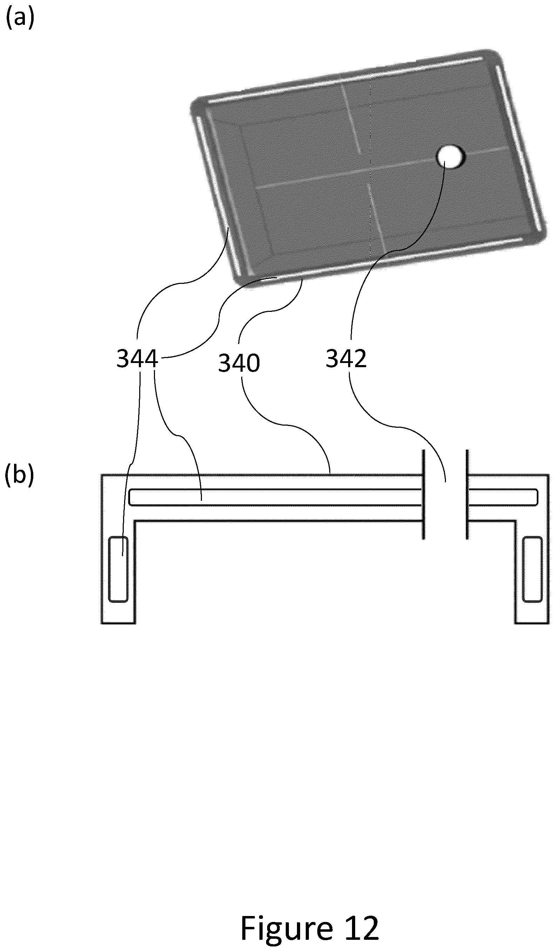

Figures 12(a) and 12(b) respectively show perspective and schematic side views of a lid jacket 340 for use with the lid 330 of Figures 8(a) and 8(b). The lid jacket 340 is configured to surround and/or cover the lid 330 so as to prevent heat transfer from the 330.

The lid jacket 340 comprises an opening 342 configured to align with the opening 332 of the lid 330.

The lid jacket 340 comprises heating elements 344. The heating elements 344 are configured to increase the temperature in the volume of air enclosed by the hydration chamber 300 between the lid 330 and the plate 310, by transferring heat through the lid 330. Additionally, or alternatively, the heat transferred from the heating elements 344 can assist in the reduction of precipitation of droplets on the interior surface of the lid 330, which serves as the ceiling for the hydration chamber during operations.

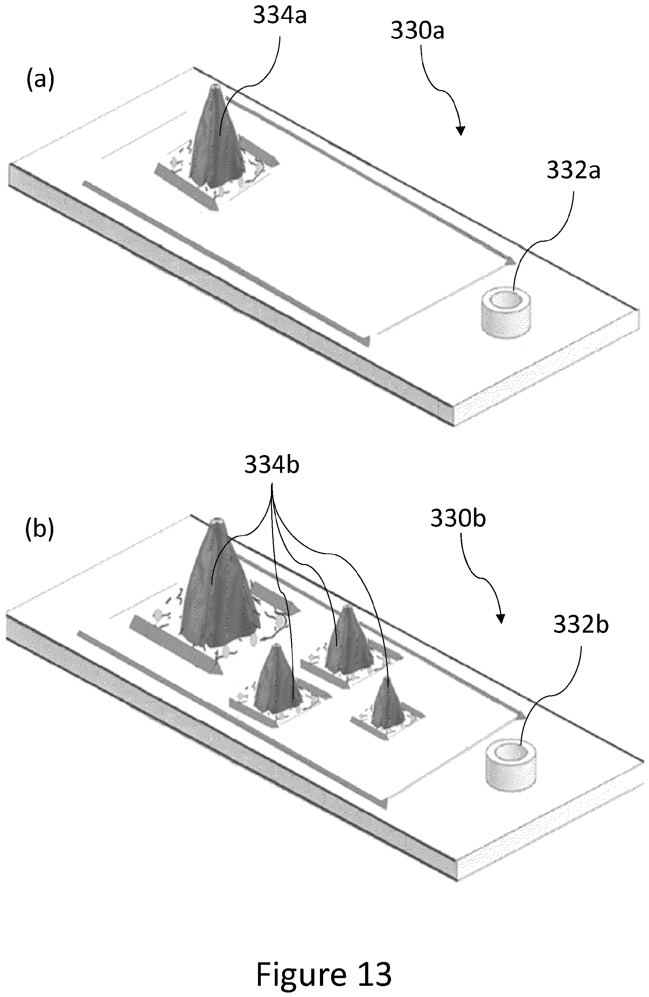

Figures 13(a) and 13(b) show perspective views of alternative lids 330a, b used in other examples of the invention.

In Figure 13(a), the lid 330a comprises an opening 332a through which liquid droplets can enter the space enclosed by the hydration chamber 300.

The lid 330a comprises a sleeve 334a. The sleeve 334a is made of, or comprises, silicone or polypropylene. In other examples, other materials which can withstand autoclaving and/or are resistant to denaturing due to repeated sterilization cycles by other chemical and ionizing methods can be used to form the sleeve 334a. The sleeve 334a is configured to enable external components, such as a robotic arm, to pass therethrough so as to enable 3D printing directly onto the plate 310 of the hydration chamber 300. As a further example, oxygen and/or media for cell growth / maintenance can be supplied via the sleeve 334a.

To assist with the 3D printing process, the lid 330a is transparent and is formed of, or comprises, polycarbonate.

The lid 330b of Figure 13(b) is substantially similar to the lid 330a of the Figure 10(a), but it comprises four sleeves 334b. The sleeves 334b of the lid 330b are differently sized so as to enable the passage of differently sized external components therethrough.

For example, a plurality of robotic arms can be inserted through the sleeves to allow simultaneous or sequential multidimensional bioprinting along multiple axes. In some examples, one of the sleeves can be used as a port for camera insertion (such as a fibre-optic camera) for an enhanced and closer visualization of 3D constructs in real-time as required.

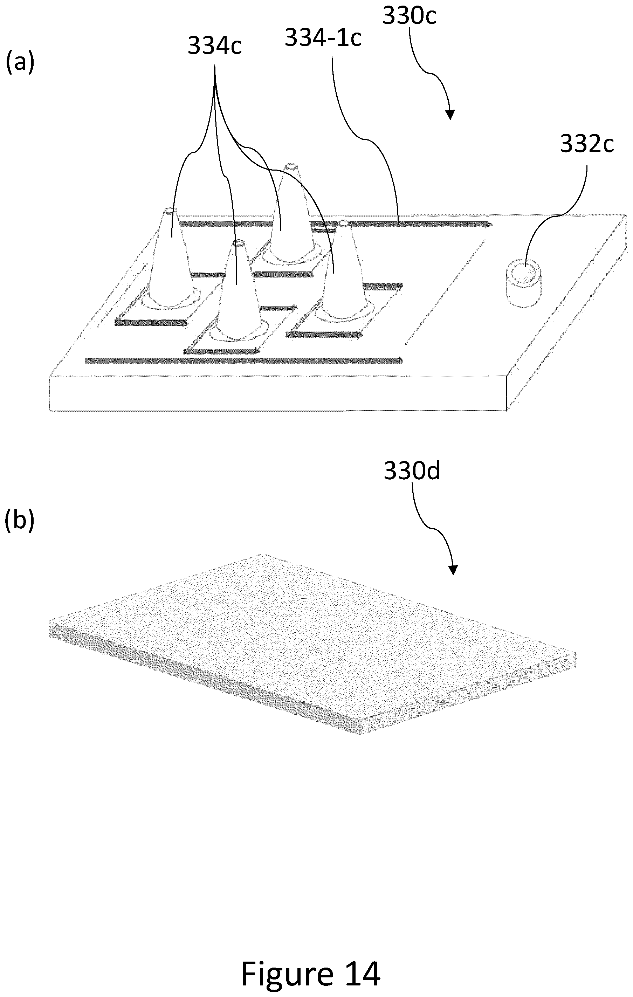

The Figures 14(a) and (b) shows perspective views of alternative lids 330c, d.

The lid 330c of Figure 14(a) enables removal of individual sleeves, or the removal of all sleeves simultaneously. Each sleeve 334c is disposed on a respective sliding plate which enables adjustment and/or removal of the individual sleeves 334c, as in figures 13(a) and 13(b). In addition, the lid 330c comprises a base plate 334-1c upon which all the sliding plates of each individual sleeve are disposed. The base plate 334-1c can be slid to remove all sleeves 334c from the lid 330c simultaneously.

The lid 330d of Figure 14(b) comprises no sleeves and no opening. This lid 330d can be used to replace any of the other lids for predetermined periods of time to prevent contamination of the 3D bioprinted structure.

Any of the lids 330 described herein can be formed of, or comprise, PTFE, PFA and/or polycarbonate.

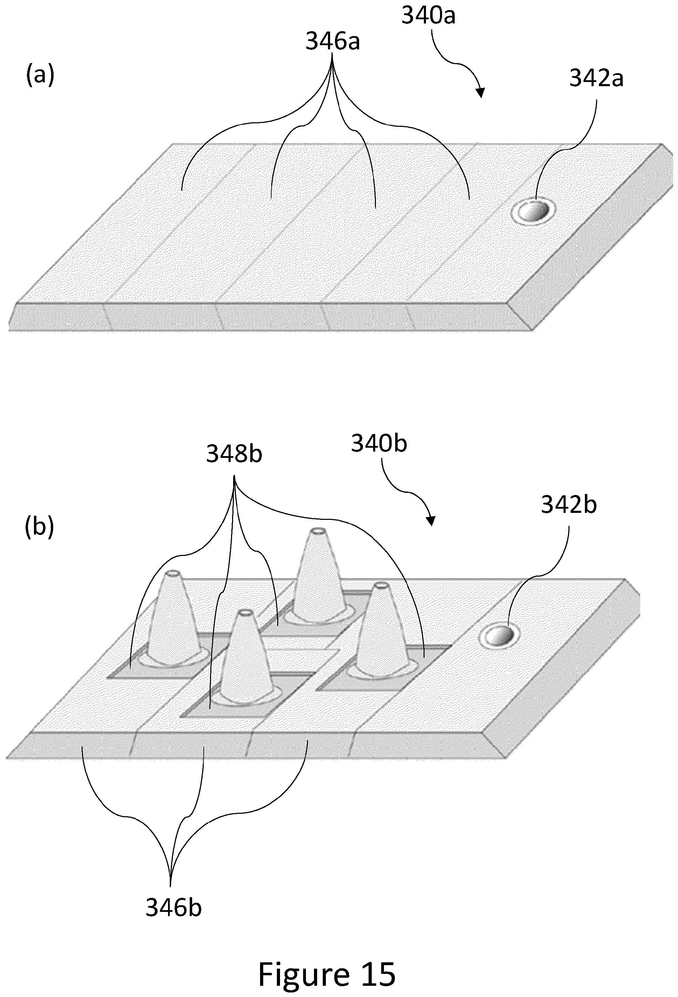

Figures 15(a) and (b) show perspective views of lid jackets 340a, b.

The lid jackets 340a, b are modular and comprise a plurality of segmented regions 346a, b into which different covers can be slot.

The modular lid jacket 340a of Figure 15(a) comprises four segmented regions 346a, and each region 346a is shown with a cover slotted therein. The covers in each region are plain and comprise no openings.

The modular lid jacket 340b of Figure 15(b) comprises three segmented regions 346b, and each region 346b is shown with a cover comprising an opening 348b. The central region 346b comprises two openings 348b, and each of the end regions 346b comprise a single opening 348b. In the example shown in Figure 15(b), sleeves from the lid below the lid jacket 340b extend through each of the openings 348b.

The modular design of the lid jackets 340a, b enables the lid jacket to be adjusted to match/accommodate the underlying lid 300. For example, the lid jacket can be configured such that openings align with each of the lid's sleeves.

Each segment of the lid jacket can be configured to function autonomously, or in synchronisation with other segments so as to provide different temperature zones, or a consistent temperature across the lid which they cover.

The example modular lids 330c, d are provided by way of example only, and the skilled person will recognise that in other examples, a combination of plain covers, and covers with any number of openings, can be added to a modular lid jacket. In other examples, each modular lid jacket can comprise any number of segmented regions.

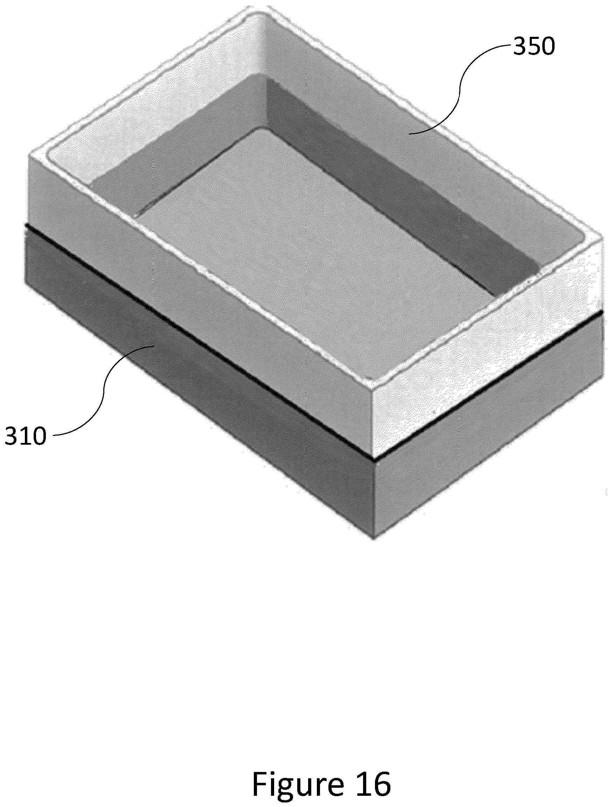

Figure 16 shows a perspective view of a wall extension 350 configured to connect to a plate 310. The wall extension 350 is a transparent wall which connects between the plate 310 and the lid 330. The wall extension 305 can be used to increase the vertical space in which the 3D bioprinting can occur. The wall extension 305 can enable a user/operator to visually monitor the printing process and/or a printed structure. In some examples, the wall extension 305 can be formed of, or comprise, polycarbonate, which can ensure that the wall extension 305 can be autoclaved/sterilized when required.

From reading the present disclosure, other variations and modifications will be apparent to the skilled person. Such variations and modifications may involve equivalent and other features which are already known in the art of hydration chambers and 3D bioprinting, and which may be used instead of, or in addition to, features already described herein.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate examples may also be provided in combination in a single example. Conversely, various features which are, for brevity, described in the context of a single example, may also be provided separately or in any suitable sub-combination. The applicant hereby gives notice that new claims may be formulated to such features and/or combinations of such features during the prosecution of the present application or of any further application derived therefrom.

For the sake of completeness, it is also stated that the term "comprising" does not exclude other elements or steps, the term "a" or "an" does not exclude a plurality, and a single processor or other unit may fulfil the functions of several means recited in the claims and any reference signs in the claims shall not be construed as limiting the scope of the claims.

## Claims

1. A device for generating liquid droplets, the device comprising:
a body configured to hold a liquid;
one or more piezoelectric actuators disposed within the body;
one or more piezoelectric sensors disposed on an underside of the body, wherein the piezoelectric sensors are configured to generate an electrical signal which is configured to activate the piezoelectric actuators;
wherein the one or more piezoelectric actuators are configured to oscillate at an ultrasonic frequency to atomise the liquid within the body to generate a plurality of droplets.

2. The device of claim 1, wherein an inner surface of the body is made of, or comprises, one or more of: PFTE, polycarbonate, PFA, and ABS.

3. The device of any of claims 1-2, wherein the piezoelectric transducers are configured to oscillate at a frequency between 0.8MHz and 2.4MHz.

4. The device of any preceding claim, further comprising a thermally insulating jacket at least partially surrounding the body; and, optionally or preferably,
further comprising a lid disposed at an end of the body, wherein the lid comprises an outlet aperture configured to receive an outlet tubing and, optionally or preferably, wherein the lid is substantially dome shaped.

5. The device of any preceding claim, further comprising a housing, wherein the housing comprises a base and an enclosure, wherein the enclosure is configured to rest on the base and wherein the body is disposed within the enclosure;
wherein the base comprises one or more side vents to enable airflow therethrough, and wherein the enclosure comprises a bottom surface comprising an opening aligned with a bottom surface of the body;
wherein the vents comprise a plurality of right-inclined struts and a plurality of left-inclined struts;
wherein the left-inclined struts are angled relative to the horizontal bottom of the base at an angle between 110-120°; and
wherein the right-inclined struts are angled relative to the horizontal bottom of the base at an angle between 40 and 60°.

6. The device of claim 5, wherein:
(i) the left-inclined struts have a diamond-shaped (i.e., rhombus) cross-section with two small angles of 30° and two large angles of 150°, and wherein a vertex with an angle of 30° directly faces outwards to receive an incoming airflow; and/or
(ii) the right-inclined struts have a diamond-shaped (i.e., rhombus) cross-section with two small angles of 60° and two large angles of 120°, and wherein the right-inclined struts are arranged such that a vertex with an angle of 60° directly faces outwards to receive an incoming airflow.

7. A system for maintaining hydration of a biostructure, the system comprising:
the device of any of claims 1-6;
a hydration chamber; the hydration chamber comprising:
a chamber lid configured to fit over a printing plate, wherein the chamber lid comprises an inlet; and
a tubing configured to convey liquid droplets generated by the device of any of claims 1-11 into the hydration chamber through the inlet.

8. The system of claim 7, wherein the hydration chamber comprises the printing plate, and optionally or preferably, wherein the printing plate is formed of, or comprises, one or more of: PFTE, PFA and polycarbonate.

9. The system of claim 7 or claim 8, wherein the chamber lid comprises an internal surface configured to face the plate, and wherein the internal surface is angled between 10-35° relative to the plate, when in use.

10. The system of any of claims 7-9, wherein the hydration chamber comprises a plate jacket configured to at least partially surround the plate, wherein the plate jacket is thermally insulating; and, optionally or preferably,
wherein the plate jacket is formed of, or comprises, polycarbonate, PFA and/or ABS.

11. The system of any preceding system claim, wherein the hydration chamber comprises a chamber lid jacket configured to at least partially surround the chamber lid, wherein the chamber lid jacket is thermally insulating; and, optionally or preferably,
wherein the chamber lid jacket is formed of a plurality of modular regions.

12. The system of any preceding system claim, wherein the plate jacket and/or the chamber lid jacket comprises one or more heating or cooling elements configured to increase or decrease the temperature of the plate.

13. The system of any preceding system claim, further comprising one or more probes configured to measure one or more environmental parameters in the space between the plate and the chamber lid.

14. The system of any preceding system claim, wherein the chamber lid comprises one or more sleeves providing access to the plate through the lid;
wherein the sleeves are made of, or comprise, silicone or polypropylene.

15. The system of any preceding claim, further comprising a wall extension disposed between the plate and the chamber lid;
wherein the wall extension is formed of, or comprises, polycarbonate; and, optionally, or preferably,
wherein the wall extension is transparent.
